(19) Europäisches Patentamt
European Patent Office
Office européen des brevets

(11) Publication number : **0 367 033 B1**

(12) **EUROPEAN PATENT SPECIFICATION**

(45) Date of publication of patent specification : **10.02.93 Bulletin 93/06**

(51) Int. Cl.$^5$ : **C07C 323/58,** C07C 323/59, C07C 319/14, A01N 37/44

(21) Application number : **89119424.3**

(22) Date of filing : **19.10.89**

(54) **S-Difluoromethylhomocysteines, preparation process, and insecticidal compositions.**

(30) Priority : **25.10.88 JP 268733/88**

(43) Date of publication of application : **09.05.90 Bulletin 90/19**

(45) Publication of the grant of the patent : **10.02.93 Bulletin 93/06**

(84) Designated Contracting States : **AT BE CH DE ES FR GB GR IT LI LU NL SE**

(56) References cited : **No relevant documents have been disclosed.**

(73) Proprietor : **SHIONOGI SEIYAKU KABUSHIKI KAISHA**
**1-8, Doshomachi 3-chome Chuo-ku**
**Osaka 541 (JP)**

(72) Inventor : **Tsushima, Tadahiko**
**1-18-13, Fushiodai**
**Ikeda-shi Osaka (JP)**
Inventor : **Ishihara, Shoichi**
**6-6-11, Mukogaoka**
**Sanda-shi Hyogo (JP)**

(74) Representative : **Vossius & Partner**
**Siebertstrasse 4 P.O. Box 86 07 67**
**W-8000 München 86 (DE)**

**EP 0 367 033 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel S-difluoromethylhomocysteines and selective insecticides containing them as an active ingredient.

Methionine is one of essential amino acids, and both L-type and D-type are effective pharmaceuticals. Methionine can be used for the treatment for hepatitis and toxicoses as a lipophilic nutrient. But no methionine derivatives showing insecticidal activity have heretofore been reported in any literature.

Aphides become frequently a subject matter of discussion as harmful insects in agriculture and gardening. Organophosphate insecticides such as malathion and pirimicarb are used as agricultural insecticides against such aphides. But the aphides possessing tolerance or resistance against these insecticides are increasing. Thus, the technical problem underlying the present invention is to provide novel compounds being effective against aphides possessing tolerance.

The present invention therefore relates to compounds of the general formula:

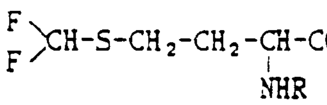

( I )

wherein R and R′ each is hydrogen or a protecting group, or acid addition salts thereof.

In this invention, a protecting group means amino- or carboxy-protecting group. Amino-protecting group means carbamate type protecting groups, such as tert-butyloxycarbonyl (BOC), p-biphenylisopropyloxycarbonyl (Bpoc), diiopropylmethoxycarbonyl (Dipmoc), or benzyloxycarbonyl (Z), aralkyl ones such as trityl or benzyl, and acyl ones such as acetyl or trifluoroacetyl. Carboxy-protecting group means alkyl such as methyl, ethyl, propyl, or butyl, aralkyl such as benzyl or benzhydryl, and pivaloyloxymethyl.

The compound of this invention can be prepared by the following Methods A and B.

Method A (Synthesis of racemate)

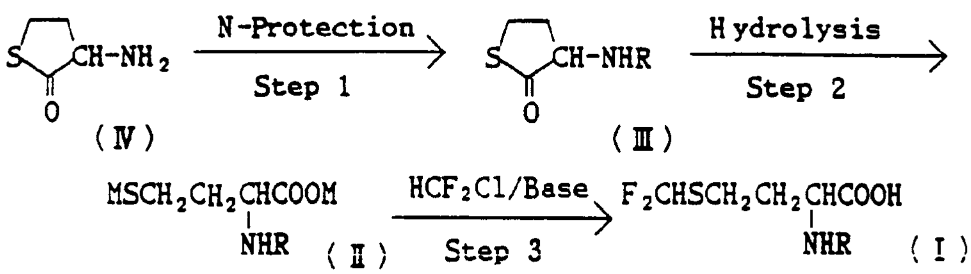


wherein M means alkali metal, and R has the same meaning as defined above.

Step 1

L-Homocysteinethiolactone (IV) is subjected to N-protecting reaction in a conventional manner. This is a process to introduce amino-protecting group such as BOC, Z, trityl, trifluoroacetyl, or acyl into the compound (IV) (M. Bodanszky and A. Bodanszky, "The Practice of Peptide Synthesis "Springer-Verlag, Tokyo, 1984, and Izumiya, et al., "Peptide Synthesis" Maruzen, Tokyo, 1985.). For the introduction of the protecting groups, BOC, trityl, p-biphenylisopropyloxycarbonyl (Bpoc), and diisopropylmethoxy carbonyl (Dipmoc), or other acid sensitive ones, the reaction is performed in an appropriate solvent at -100 to 50 °C, preferably at -50 °C to room temperature. As reagents for introducing BOC, there are exemplified di-tert-butyl dicarbonate, tert-butyloxy-carbonyl chloride, tert-butyloxycarbonyl azide, tert-butyl-4,6-dimethylpyrimidyl-2-thiolcarbonate, and 2-(tert-butoxycarbonyloxyimino)-2-phenylacetonitrile. Trityl chloride can be used for introducing trityl. If necessary, a base can be added. As the base there are exemplified organic bases such as triethylamine, pyridine, or the like, and inorganic bases such as sodium hydrogen carbonate, sodium carbonate, sodium hydroxide or the like. As the solvent there are exemplified alcohols, ethers, methylene chloride, chloroform etc. These amino-protecting groups have some advantages for avoiding racemization and for facile acidic cleaveage. When the protecting group is Z group, the compound (IV) is reacted with a reagent such as benzyloxycarbonyl chloride in an appropriate solvent in the presence of a base at 0 to 100 °C, preferably at a temperature around room

temperature. As the base used for the reaction there are exemplified organic bases such as triethylamine or the like and inorganic bases such as sodium hydroxide, sodium hydrogencarbonate or the like. The same solvent mentioned above can be used, too.

Step 2

N-Protected-homocysteinethiolactone (III) obtained above is subjected to hydrolysis to give the dialkali metal salt (II). This reaction is performed in an appropriate solvent at 0 to 100 °C for several minutes to several hours. As alkali used for the reaction there are exemplified sodium bicarbonate, potassium carbonate, potassium t-butoxide, and sodium hydride. As the solvent used for the reaction there are exemplified water, alcohols, or aqueous alcohols.

Step 3

Alkali metalic salt (II) obtained above is reacted with chlorodifluoromethane in the presence of a base to give the objective compound (I). Difluoromethylation of thiols are documented in J. Hine et al, J. Am. Chem. Soc., 79, 5493 (1957); T. Tsuji et al, J. Antibiotics, X X X VIII, 466 (1985); M. Hudlicky, "Chemistry of Organic Fluorine Compounds," 2nd. Ed., Ellis Horwood Ltd., Chichester, England, 1972, p444.. But these methods are restricted only to compound having simple functional groups, and there is no report that said methods have been applied to amino acids or compounds possessing the free carboxy group. Accordingly it is to be emphasized that novel difluoromethylhomocysteine (I) can be conveniently prepared hereby in good yield. The product (I) can be subjected to N-deprotection of amino group and/or esterification of carboxy group, if necessary. When the N-protecting group is benzyloxycarbonyl, it can be removed by hydrogenation or reacting with HBr-/AcOH in a conventional manner. When the protecting group is BOC, it is removed by reacting with HBr/AcOH, trifluoroacetic acid/organic solvent (e.g. ethers, methylene chloride, chloroform, etc.), or HCl/organic solvent (e.g. alcohols, methylene chloride, chloroform, etc.). When the protecting group is trityl, it is easily removed by treatment with acids such as acetic acid, trifluoroacetic acid, formic acid, hydrochloric acid, etc. Further, the esterification of carboxy is carried out in an appropriate solvent in a conventional manner, for example, acid-alcohol, diazo compound, thionyl chloride-alcohol, acid halide-base-alcohol or the like. As the solvent, there are exemplified alcohols, THF (tetrahydrofuran), ether, and methylene chloride, etc. The reaction is performed at a temperature -20 to 100 °C, preferably at a temperature 0 °C to room temperature. The compound (I) of the present invention obtained in this method, however, is a complete racemate. Even if as a starting material was used an optically active L-homocysteine thiolactone (IV), the resulting product is a racemate in this method. It is caused by the racemization taking place in Step 2. Thus, the present inventors have investigated catalytic hydrolysis using Ag, Hg, and Cu salts as catalysts [S. Masamune, et al., J. Am. Chem Soc., 97, 3515 (1975)] for avoiding racemization in Step 2, but these attempts failed. Thus, development of another method for preparing such an optically active substance will be shown below as Method B.

Method B (Asymmetric synthesis of L-form)

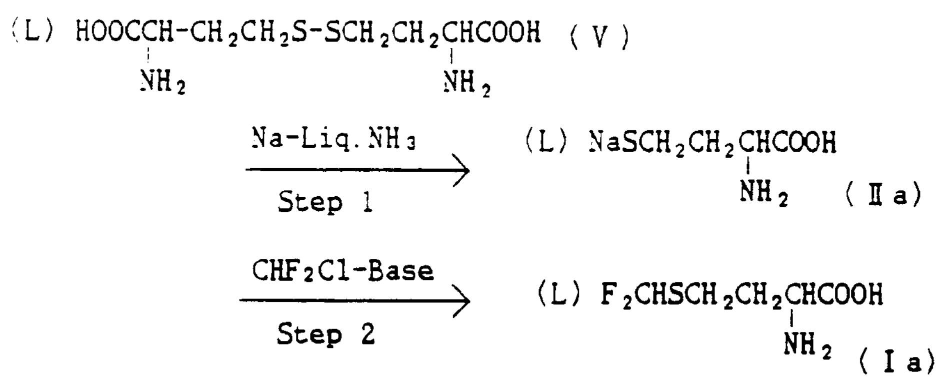


(V) (IIa) (Ia)

Step 1

This step is directed to a process for preparing L-homocysteine sodium salt (IIa) by subjecting L-homocystine (V) to liquid ammonium-sodium reduction in a conventional manner (K. Ramalingam and R. W. Woodard, J. Org. Chem., 1984, 49, 1291.). L-Homocysteine sodium salt (IIa), which is a useful intermediate for the

chemistry of peptides, can be obtained without racemization by this reaction. It is recommended that isolation, purification, or preservation of the intermediate (IIa) would be carried out under anaerobic conditions, preferably under nitrogen atmosphere, because of its susceptibility to decomposition in the air (due to $CO_2$ or $O_2$).

Step 2

The compound (IIa) obtained in Step 1 is reacted with chlorodifluoromethane in the presence of a base to give the compound (I a) of the present invention. Thus, it has been found that optically active L-difluromethylhomocysteine (Ia) can be prepared at one step without racemization in this reaction. Accordingly, the reaction is considered as excellent diastereo-selective synthesis of the product (Ia). It is recommended that the reaction is performed by carefully avoiding the introduction of air.

The following examples, reference examples and formulation are shown to clarify the practical embodiments of this invention.

Example 1

A solution of 1.38 g (6.35 mmol) of N-tert-butyloxycarbonyl-homocysteinethiolactone (III) in 8 ml of ethanol is mixed with 6.67 ml of 2N-NaOH. The mixture is stirred at room temperature for 1 hour and concentrated to give white powder. It is dried in a desiccator for 2 days. The resulting powder (II) (279 mg, 1 mmol) is dissolved in 2 ml of ethanol and mixed with chlorodifluoromethane at room temperature under stirring. The mixture is stirred for 8 hours, neutralized with 1N-HCl under ice-cooling, and concentrated under reduced pressure. The residue is basified with 0.5N-NaOH and washed with ether. The washing is acidified to pH 2.0 with 1N-HCl, and the mixture is salted out and extracted three times with ethyl acetate. The organic layer is washed twice with saturated brine. And the solution is dried over magnesium sulfate and concentrated under reduced pressure to give N-BOC-difluoromethylhomocysteine (I-1) as an oily substance.

$^1$HNMR $\delta$ ($CDCl_3$) :

1.43 (s, 9H, t-Bu); 1.80-2.40 (m, 2H); 2.45-3.00 (m, 2H); 4.40 (br.m, 1H); 5.33 (br.m, 1H); 6.8 (t, 1H, J=5.7Hz)

The compound obtained above is converted into benzhydryl ester and chromatographed on a column of silica gel HPLC (Lobar B®) eluting with benzene/ethyl acetate (4/1 v/v) to give 269 mg (Yield : 59.6 %) of the ester compound.

The compound obtained above is deprotected in trifluoroacetic acid/anisole to give objective amino compound. It is subjected to desalting with Dowex-50W X8 to give 77 mg (Yield : 43 %) of (DL)-difluoromethylhomocysteine. mp. >195 °C (dec.)

$[\alpha]_D^{26}$ 0±0.4° (c, 1.002, 4N-HCl)

Example 2

Into a solution of 2.04 g (10.56 mmol) of L-homocysteine sodium salt in 18 ml of ethanol is vigorously bubbled chlorodifluoromethane at room temperature while keeping the dropwise addition of potassium tert-butoxide in ethanol (2.03 g, 2.01 mmol/12 ml) placed in funnel over 1 to 2 hours. After the addition of the base, chlorodifluoromethane is introduced for additional 1 hour and then the solution is acidified (pH 4.3) with 1N HCl under ice-cooling and concentrated under reduced pressure. The residue is well dried by distilling off the freshly added ethanol twice and again 200 ml of methanol added to precipitate inorganic salts. The insoluble salts are filtered off and the filtrate is concentrated to give 1.7 g of the residue. The residue is purified by column chromatography successively on ion exchange resin Dowex 50 W-X8 using 1N-$NH_3$ as an eluent, and on HP-20 using an aqueous methanol as an eluent to give 1.1 g (Yield : 56 %) of the desired compound L-difluoromethylhomocysteine. mp. >195 °C (dec.)

$[\alpha]_D^{26}$+23.4±0.8° (c, 0.822, 4N-HCl)

$^1$HNMR ($D_2O$, 200 MHz) $\delta$ :

2.33 (m, 2H, ß-C$\underline{H}$), (t, 2H, J=7.8Hz, $\gamma$-C$\underline{H_2}$); 3.84 (t, 1H, J=6.4 Hz, $\alpha$-C$\underline{H_2}$); 7.07 (t, 1H, J=55.8 Hz, C$\underline{HF}_2$)

IR (KBr):

3400 (m); 3200-2400 (br,s); 1585 (br, s); 1513 (s); 1408 (s); 1326 (m); 1015 (s); 765 (m) $cm^{-1}$

Mass m/z:

186 ($MH^+$), 167 ($MH^+$-F), 140 ($M^+$-COOH), 134 ($M^+$-$CHF_2$), 111 ($F_2CHSCH_2CH_2$), 97 ($F_2CHSCH_2$), 74 ($M^+$-$F_2$ $CHSCH_2CH_2$)

Anal Calcd. (%) for $C_5H_9NF_2S$

C, 32.43; H, 4.90; N, 7.56; F, 20.52; S, 17.31

Found : C, 32.31; H, 4.87; N, 7.54; F, 20.21; S, 17.60

## Effect of the Invention

1. Test Method

### Preparation of a test solution

A solution of the compound is diluted with distilled water containing Tween 20® at a concentration of 100 ppm to prepare a predetermined concentration of the test solution. Malathion emulsion and pirimicarb wettable powder are diluted with distilled water to prepare a predetermined concentration of the test solution, respectively.

### Aphides tested

Myzus persicae (URR-O type) distributed from Chiba University, which were reared for succesive generations, were used as a group of sensitive strains. Myzus persicae resistant to malathion and pirimicarb which were collected from Aburahi Laboratory of Shionogi & Co., Ltd. and reared for succesive generations while selecting those resistant to these compounds were used as a group of resistant strains.

### Experiment

A polyethylene cup (diameter 6 cm, depth 4 cm) was filled with 0.3 % agar gel and a piece of Chinese cabbage leaf (3x3 cm) was placed on the gel. One apterous adult of Myzus persicae was put on the leaf and allowed to keep at 25 °C for 24 hours to make larviposition. After removal of the adult, 2 ml of the test solution was applied to the foliage under a rotary sprayer; and larvae of Myzus persicae was allowed to keep at 25 °C for 48 hours. Then the mortality of the larvae was determined and the value of $LC_{50}$ was calculated by the method of Billis. [Billis, Ann. Appl. Biol.,22, 134;307 (1935)].

Result

Table 1

Mortality (%) against Myzus persicae

| Compound | Concentration (ppm) | Mortality (%) | |
|---|---|---|---|
| | | Sensitive Strain | Resistant Strain |
| Compound I a *1 | 250 | 100 | 100 |
| | 125 | 100 | 100 |
| | 62.5 | 57.1 | 100 |
| | 31.3 | 4.8 | 54.2 |
| | 15.6 | 8.7 | 0.0 |
| | 7.8 | 0.0 | 5.0 |

*1 : L-Difluoromethylhomocysteine

Table 2

The value of $LC_{50}$ against Myzus persicae

| Test Compound | $LC_{50}$ (ppm) | |
|---|---|---|
| | Sensitive Strain | Resistant Strain |
| Compound I a | 52.9 (48.1-58.3)*2 | 27.1 (24.8-29.7) |
| malathion | 136.0 (122.7-150.8) | 2000 < |
| pirimicarb | 8.2 (7.5-9.0) | 1920 < |

*2 : ( ) means 95 % confidence limit

## Claims

### Claims for the following Contracting States : AT, BE, CH, DE, FR, GB, GR, IT, LI, LU, NL, SE

1. Compounds of the general formula:

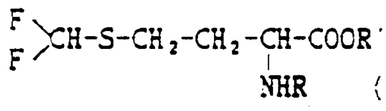

( I )

wherein R and R′ each is hydrogen or a protecting group, or acid addition salts thereof.

2. The compounds claimed in claim 1, in the optically active L-form.

3. The compounds claimed in claim 2, wherein R and R′ each is hydrogen.

4. A process for preparing the compounds claimed in claim 1 which comprises subjecting a compound of the general formula:

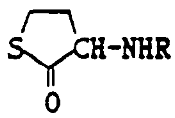

(III)

to alkaline hydrolysis to form a compound of the general formula:

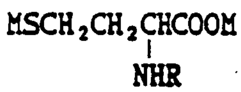

(II)

wherein M is an alkali metal cation and R has the same meaning as defined in claim 1 and reacting said compound with chlorodifluoromethane in the presence of a base, and if necessary, followed by removing the protective group.

5. A process for preparing the compounds claimed in claim 3 which comprises reacting L-homocysteine sodium salt prepared from L-homocystine without racemization with chlorodifluoromethane in the presence of a base.

6. Insecticidal composition comprising a compound claimed in claim 1 as an active ingredient.

### Claims for the following Contracting State : ES

1. A process for preparing the compounds of the general formula:

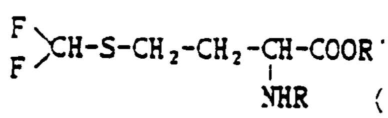

( I )

wherein R and R′ each is hydrogen or a protecting group, or acid addition salts thereof which comprises subjecting a compound of the general formula:

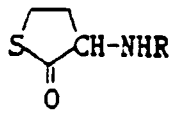

(III)

to alkaline hydrolysis to form a compound of the general formula:

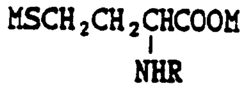

(II)

wherein M is an alkali metal cation and R has the same meaning as defined in claim 1 and reacting said compound with chlorodifluoromethane in the presence of a base, and it necessary, followed by removing the protective group.

2. Process as claimed in claim 1, wherein the compound obtained are in the optically active L-form.

3. A process for preparing the compounds obtained according to claim 2 wherein R and R′ each is hydrogen, which comprises reacting L-homocysteine sodium salt prepared from L-homocystine without racemization with chlorodifluoromethane in the presence of a base.

4. Insecticidal composition comprising a compound obtained according to claim 1 as an active ingredient.

**Patentansprüche**

**Patentansprüche für folgende Vertragsstaaten : AT, BE, CH, DE, FR, GB, GR, IT, LI, LU, NL, SE**

1. Verbindungen der allgemeinen Formel:

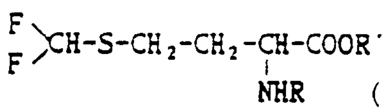

( I )

in der R und R′ jeweils ein Wasserstoffatom oder eine Schutzgruppe bedeuten, oder deren Säureadditions-Salze.

2. Verbindungen nach Anspruch 1 in der optisch aktiven L-Form.

3. Verbindungen nach Anspruch 2 wobei R und R′ jeweils ein Wasserstoffatom sind.

4. Verfahren zur Herstellung der Verbindungen nach Anspruch 1, umfassend die alkalische Hydrolyse einer Verbindung der allgemeinen Formel:

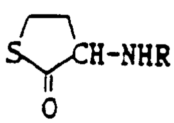

(III)

zu einer Verbindung der allgemeinen Formel:

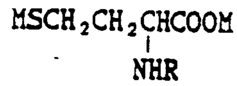

(II)

in der M ein Alkalimetallkation ist und R die in Anspruch 1 angegebene Bedeutung hat, und Umsetzung dieser Verbindung mit Chlordifluormethan in Gegenwart einer Base und gegebenenfalls das Entfernen der Schutzgruppe.

**5.** Verfahren zur Herstellung der Verbindungen nach Anspruch 3, umfassend die Umsetzung des Natriumsalzes von L-Homocystein, hergestellt aus L-Homocystin, mit Chlordifluormethan in Gegenwart einer Base ohne Racemisierung.

**6.** Insektizide Zusammensetzung, enthaltend eine Verbindung nach Anspruch 1 als Wirkstoff.

**Patentansprüche für folgenden Vertragsstaat : ES**

**1.** Verfahren zur Herstellung von Verbindungen der allgemeinen Formel:

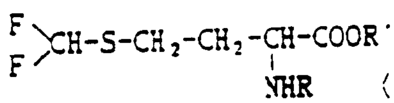

( I )

in der R und R′ jeweils ein Wasserstoffatom oder eine Schutzgruppe bedeuten, oder deren Säureadditions-Salze, umfassend die alkalische Hydrolyse einer Verbindung der allgemeinen Formel:

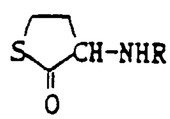

(III)

zu einer Verbindung der allgemeinen Formel:

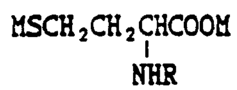

(II)

in der M ein Alkalimetallkation ist und R die vorstehende Bedeutung hat, und Umsetzung dieser Verbindung mit Chlordifluormethan in Gegenwart einer Base und gegebenenfalls das Entfernen der Schutzgruppe.

**2.** Verfahren nach Anspruch 1, wobei die erhaltenen Verbindungen in der optisch aktiven L-Form sind.

**3.** Verfahren zur Herstellung der nach Anspruch 2 erhaltenen Verbindungen, wobei R und R′ jeweils ein Wasserstoffatom sind, dadurch gekennzeichnet, daß die Umsetzung des Natriumsalzes von L-Homocystein, hergestellt aus L-Homocystin, mit Chlordifluormethan in Gegenwart einer Base ohne Racemisierung erfolgt.

**4.** Insektizide Zusammensetzung, die eine nach Anspruch 1 erhaltene Verbindung als Wirkstoff enthält.

## Revendications

### Revendications pour les Etats contractant suivants : AT, BE, CH, DE, FR, GB, GR, IT, LI, LU, NL, SE

1. Composés de la formule générale :

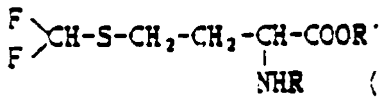

( I )

dans laquelle R et R′ représentent chacun de l'hydrogène ou un groupe de protection, ou leurs sels d'addition d'acide.

2. Composés suivant la revendication 1 sous la forme L optiquement active.

3. Composés suivant la revendication 2, caractérisés en ce que R et R′ représentent chacun de l'hydrogène.

4. Procédé de préparation des composés suivant la revendication 1, qui consiste à soumettre un composé de la formule générale :

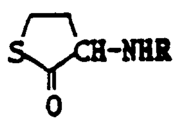

(III)

à une hydrolyse alcaline pour former un composé de la formule générale :

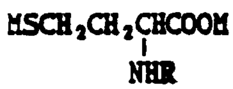

(II)

dans laquelle M représente un cation de métal alcalin et R a la même signification que donnée dans la revendication 1, et à faire réagir ce composé avec du chlorodifluorométhane en présence d'une base, et si nécessaire, à enlever ensuite le groupe protecteur.

5. Procédé de préparation des composés suivant la revendication 3, qui consiste à faire réagir du sel de sodium de L-homocystéine préparé à partir de L-homocystine sans racémisation avec du chlorodifluorométhane en présence d'une base.

6. Composition insecticide comprenant un composé suivant la revendication 1 comme ingrédient actif.

### Revendications pour l'Etat contractant suivant : ES

1. Procédé de préparation des composés de la formule générale :

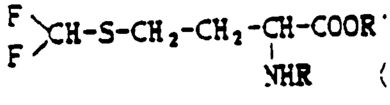

( I )

dans laquelle R et R′ représentent chacun de l'hydrogène ou un groupe de protection, ou leurs sels d'addition d'acide, qui consiste à soumettre un composé de la formule générale :

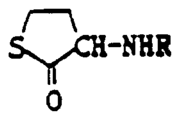 (III)

à une hydrolyse alcaline pour former un composé de la formule générale :

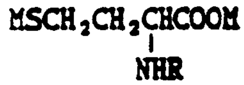 (II)

dans laquelle M représente un cation de métal alcalin et R a la même signification que donnée précédemment et à faire réagir ce composé avec du chlorodifluorométhane en présence d'une base, et si nécessaire, à enlever ensuite le groupe protecteur.

**2.** Procédé suivant la revendication 1, caractérisé en ce que les composés obtenus sont sous la forme L optiquement active.

**3.** Procédé de préparation des composés obtenus suivant la revendication 2, caractérisé en ce que R et R′ représentent chacun de l'hydrogène, qui consiste à faire réagir du sel de sodium de L-homocystéine préparé à partir de L-homocystine sans racémisation avec du chlorodifluorométhane en présence d'une base.

**4.** Composition insecticide comprenant un composé obtenu suivant la revendication 1 comme ingrédient actif.